**Europäisches Patentamt**

(19) **European Patent Office**

**Office européen des brevets**

(11) Veröffentlichungsnummer: **0 020 959**
**B1**

(12) # EUROPÄISCHE PATENTSCHRIFT

(45) Veröffentlichungstag der Patentschrift:
29.06.83

(21) Anmeldenummer: 80102486.0

(22) Anmeldetag: 07.05.80

(51) Int. Cl.³: **C 07 D 475/14** // C07C59/105,
C07H3/02, C07C91/10,
C07C107/06, C12P7/58

(54) **Verfahren zur Herstellung von Riboflavin.**

(30) Priorität: 08.06.79 DE 2923266
08.06.79 DE 2923267
08.06.79 DE 2923268
06.02.80 DE 3004304

(43) Veröffentlichungstag der Anmeldung:
07.01.81 Patentblatt 81/1

(45) Bekanntmachung des Hinweises auf die Patenterteilung:
29.06.83 Patentblatt 83/26

(84) Benannte Vertragsstaaten:
AT BE CH DE FR GB IT LI NL SE

(56) Entgegenhaltungen:
US-A-2 350 376
US-A-2 429 244
US-A-2 477 560

JOURNAL OF THE AMERICAN CHEMICAL SOCI-
ETY, Band 81, Nr. 19, Oktober 1959, Washington, US,
R.L. WHISTLER et al.: "Preparation of D-arabinose
from D-glucose with Hypochlorite", Seiten 5190–5192

CHEMICAL ABSTRACTS, Band 77, Nr. 19,
6. November 1972, Zusammenfassung Nr. 126 942s,
Seite 430, Columbus, Ohio, US, V. BILIK: "Reactions of
saccharides catalyzed by molybdate ions. IV. Epimerization of aldopentoses"

CHEMICAL ABSTRACTS, Band 55, Nr. 13, 26. Juni
1961, Zusammenfassung Nr. 12 307b, Columbus, Ohio,
US, V.M. BEREZOVSKII et al.: "Preparation of 3,4-xy-
lyl-N-d-ribitylamine from a mixture of d-ribose and d-
arabinose"

(73) Patentinhaber: Merck Patent Gesellschaft mit
beschränkter Haftung, Frankfurter Strasse 250,
D-6100 Darmstadt (DE)

(72) Erfinder: Wolf, Rudolf, Dr., Moserstrasse 6,
D-6100 Darmstadt (DE)
Erfinder: Reiff, Fritz, Dr., im Rassdorf 6, D-6104 Seeheim
(DE)
Erfinder: Wittmann, Rolf, Dr., Tannenstrasse 6,
D-6109 Mühltal (DE)
Erfinder: Butzke, Jürgen, Waldstrasse 48,
D-6110 Dieburg (DE)

## Verfahren zur Herstellung von Riboflavin

Die Erfindung betrifft ein neues Verfahren zur Herstellung von Riboflavin, ausgehend von D-Glucose.

Verfahren zur Herstellung von Riboflavin sind bekannt. Das üblicherweise grosstechnisch durchgeführte Verfahren geht von D-Glucose aus, die zu D-Arabonsäure oxidiert wird, die zu D-Ribonsäure epimerisiert und dann zum D-Ribonolacton umgesetzt wird, aus dem durch Amalgamreduktion D-Ribose gebildet wird, die über das Xylidinribosid zum N-D-Ribityl-3,4-xylidin hydriert wird. Aus dem N-D-Ribityl-3,4-xylidin kann dann durch Umsetzung mit diazotiertem Anilin und Barbitursäure Riboflavin gewonnen werden. Dieses Verfahren ist beschrieben in Kirk-Othmer, Band 17, S. 451, sowie in Chemical Abstracts, Band 55, Zusammenfassung Nr. 12 307 b.

Die Ausbeute bei diesem Verfahren liegt für die Stufen von D-Glucose bis N-D-Ribityl-3,4-xylidin bei etwa 20–23%. Daraus ergibt sich eine Gesamtausbeute von Riboflavin, ausgehend von D-Glucose, von 15–16% der Theorie. Nachteilig neben der relativ schlechten Gesamtausbeute ist bei diesem Verfahren auch die Epimerisierungsstufe von der D-Arabonsäure zur D-Ribonsäure, bei der eine Reihe von harzigen Nebenprodukten gebildet werden, die eine komplizierte Aufreinigung erforderlich machen. Insbesondere bereitet jedoch die Amalgamreduktion sehr grosse Probleme, da beim Arbeiten mit grossen Mengen Quecksilber erhebliche Anstrengungen gemacht werden müssen, um sowohl die Produkte als auch die Abfallstoffe frei von Quecksilber zu halten.

Es sind deshalb schon Verfahren vorgeschlagen worden, die das Arbeiten mit Quecksilber vermeiden. So kann D-Ribonsäure oder D-Ribonolacton in Gegenwart von 3,4-Xylidin oder 4-Nitro-1,2-xylol in einer Stufe zum N-D-Ribityl-3,4-xylidin hydriert werden (siehe z.B. DE-B-2 558 515, DE-B-2 558 516, DE-B-2 650 232 und DE-B-2 650 830). Dies erfordert jedoch eine sehr investitionsintensive Hochdruckhydrieranlage, da die Hydrierung bei einem Druck von 250–300 bar abläuft. Trotz der Einsparung von Zwischenstufen und trotz der hohen Aufwendungen liegt bei diesem Verfahren die Ausbeute an N-D-Ribitylxylidin ausgehend von D-Glucose auch nur bei etwa max. 35%, woraus sich eine Riboflavinausbeute von weniger als 25% ergibt.

Es ist weiterhin vorgeschlagen worden, D-Ribose direkt durch Fermentation in einem mikrobiologischen Prozess mit Hilfe von geeigneten Mikroorganismen aus D-Glucose herzustellen und daraus dann in üblicher Weise N-D-Ribityl-xylidin und Riboflavin zu gewinnen (siehe DE-B-1 904 265 und DE-A-2 454 931). Obwohl dieser Weg Vorteile aufweist, sind doch bei der grosstechnischen Durchführung des komplizierten und vor allem störanfälligen biochemischen Prozesses erhebliche Schwierigkeiten zu erwarten, die die Wirtschaftlichkeit des Verfahrens beeinträchtigen.

Darüber hinaus liegt auch bei diesem Verfahren die Ausbeute an N-D-Ribityl-xylidin, ausgehend von D-Glucose, nur bei etwa 34%, woraus sich eine Riboflavinausbeute von etwa 24% ergibt.

Es bestand deshalb die Aufgabe, ein Verfahren zur Herstellung von Riboflavin zu finden, das in einfachen, ohne grosse Investitionen durchzuführenden Reaktionsschritten unter Verwendung preiswerter Ausgangsmaterialien und unter Vermeidung umweltschädlicher, hochgiftiger Reaktionskomponenten in guter Ausbeute zu einem reinen Produkt führt. Diese Aufgabe wurde durch die vorliegende Erfindung gelöst.

Es wurde nämlich gefunden, dass überraschenderweise ein völlig neuartiger Weg, der ebenfalls von D-Glucose ausgeht, jedoch nicht über Arabonsäure und Ribonsäure führt, sondern über Gluconsäure und Arabinose D-Ribose und N-D-Ribityl-3,4-xylidin liefert, die Nachteile der bekannten Verfahren vermeidet und sogar mit höheren Ausbeuten als die bekannten Verfahren zu Riboflavin führt. Dies ist insofern überraschend, als die Einzelschritte des erfindungsgemässen Verfahrens als solche jeweils bekannt waren. Es konnte jedoch nicht vorausgesehen werden, dass gerade diese Kombination von Einzelreaktionen zu einem so vorteilhaften Gesamtergebnis führen würde, zumal zahlreiche Versuche gemacht worden sind, dieses wichtige Produkt auch in grösserem Massstab vorteilhaft herzustellen. Insbesondere konnte nicht vorhergesehen werden, dass durch erfinderische Verbesserungen zahlreicher Einzelschritte dieser Kombination das schon sehr vorteilhafte Gesamtkonzept noch verbessert werden konnte.

Gegenstand der Erfindung ist demgemäss ein Verfahren zur Herstellung von Riboflavin, das dadurch gekennzeichnet ist, dass man aufeinanderfolgend

a) D-Glucose zu D-Gluconsäure bzw. einem Alkaligluconat oxidiert,

b) das so erhaltene Gluconat mit Hilfe von Hypochlorit in D-Arabinose überführt,

c) D-Arabinose mit einer Molybdän(VI)-Verbindung in D-Ribose umwandelt,

d) D-Ribose in Gegenwart von Nitroxylol oder Xylidin hydriert,

e) das erhaltene N-D-Ribityl-3,4-xylidin durch Behandlung mit dem entsprechenden Diazoniumsalz zu 1-D-Ribitylamino-3,4-dimethyl-6-phenylazobenzol umsetzt und

f) diese Azoverbindung durch Umsetzung mit Barbitursäure in Riboflavin überführt.

Überraschend vorteilhaft bei diesem Verfahren ist, dass die einzelnen Stufen ohne Schwierigkeiten mit preiswerten und ungiftigen Reaktionskomponenten durchgeführt werden können und dass zum Teil auf eine Isolierung von Zwischenprodukten ohne Ausbeuteverluste verzichtet werden kann, so dass man ohne grossen Arbeits- und Investitionsaufwand zum Endprodukt gelangt.

Überraschend ist insbesondere, dass trotzdem sehr gute Ausbeuten erzielt werden, die über denjenigen liegen, die bei den bisher bekannten Verfahren erzielt werden. So wird nach dem erfindungsgemässen Verfahren N-D-Ribitylxylidin, ausgehend von D-Glucose, in einer Ausbeute von etwa 40% gewonnen, was einer Riboflavinausbeute von fast 30%, bezogen auf D-Glucose, entspricht.

Zur Durchführung des Verfahrens kann wie bei den bekannten Verfahren von der gut zugänglichen, preiswerten D-Glucose ausgegangen werden. D-Gluconsäure ist daraus in sehr hohen Ausbeuten von über 90% in bekannter Weise durch fermentative, chemische oder elektrochemische Oxidation zu erhalten. Eine Übersicht über solche Verfahren findet sich z.B. in Ind. Eng. Chem. 11, S. 370–372 (1972).

Bevorzugt wird die fermentative Oxidation durchgeführt. Dazu wird D-Glucose in wässeriger Lösung unter Zusatz von Nährstoffen in einem Fermenter unter sterilen Bedingungen mit einem geeigneten Bakterienstamm wie z.B. Acetobacter suboxydans unter Belüften und Neutralisieren der entstehenden Säure mit einer Base, vorzugsweise Natronlauge, so lange behandelt, bis der Test auf Zucker negativ ausfällt, d.h. die gesamte Glucose umgesetzt ist. Je nach Ansatzgrösse und Bakterienstamm ist die Oxidation in etwa 10 bis 40 Stunden beendet. Die dabei in praktisch quantitativer Ausbeute anfallende, etwa 20%ige Gluconat-Lösung kann unmittelbar zur Weiteroxidation zu Arabinose verwendet werden.

Überraschenderweise wurde gefunden, dass die an sich bekannte Oxidation von Gluconat (vorzugsweise Alkaligluconat) zu Arabinose mit Hypochlorit wesentlich verbessert werden kann, wenn man die Oxidation mit hoher Gluconatkonzentration und bei hoher Temperatur durchführt. Dies ist überraschend, denn es war zwar aus dem J. Amer. Chem. Soc., Band 81, Seite 5190 (1959) bekannt, dass Gluconat mit Natriumhypochlorit zu Arabinose oxidiert werden kann. Dabei wird jedoch wegen der Instabilität des Hypochlorits nur in sehr verdünnter Lösung und bei relativ niedrigen Temperaturen gearbeitet. Die Oxidation, zu der etwa das 2,5fache der theoretischen Menge an Hypochlorit eingesetzt wird, läuft dabei über etwa 20–30 Stunden und liefert nur eine Ausbeute von etwa 40%.

Überraschenderweise kann bei dem erfindungsgemässen Verfahren durch die Verwendung von erhöhten Temperaturen nicht nur eine doppelt so hohe Ausbeute erzielt werden, sondern man kann darüber hinaus auch bei sehr viel höheren Konzentrationen arbeiten. Da auch die Reaktionszeit von etwa 20–30 Stunden bei dem erfindungsgemässen Verfahren auf etwa 10–60 Minuten verkürzt werden kann, wird dem erfindungsgemässen Verfahren eine um Zehnerpotenzen verbesserte Raum/Zeit-Ausbeute erreicht. Dies ist besonders deshalb überraschend, weil an sich Arabinose unter diesen Verfahrensbedingungen in schneller Reaktion mit Hypochlorit weiter oxidiert bzw. abgebaut wird. Trotz dieser einen schnellen Umsatz und grosstechnischen Durchsatz ermöglichenden Bedingungen wird bei dem erfindungsgemässen Verfahren sogar noch das Oxidationsmittel sehr viel besser ausgenutzt als bei der vorbekannten Reaktion. Bei der erfindungsgemässen Reaktion werden nämlich nur etwa 1,0–1,5, in der Regel etwa 1,1–1,2 Äquivalente des Oxidationsmittels benötigt gegenüber 2,5 Äquivalenten bei der bekannten Reaktion.

Um diese überraschend vorteilhaften Verfahrensbedingungen erreichen zu können, ist es keineswegs nötig, besonders reine Ausgangsmaterialien zu verwenden. Das als Ausgangsmaterial verwendete Natriumgluconat kann in sehr guter Ausbeute in üblicher Weise durch Fermentation oder durch chemische oder elektrochemische Oxidation aus D-Glucose gewonnen werden. Es ist ohne weiteres möglich und wird auch vorteilhafterweise so gehandhabt, die dabei anfallende Gluconatlösung direkt einzusetzen. Da z.B. bei der bekannten Fermentation von Glucose eine etwa 20–35%ige Gluconat-Lösung anfällt, wird vorteilhafterweise direkt mit dieser Lösung weitergearbeitet, obwohl an sich auch höher konzentrierte Lösungen zu verwenden sind.

Diese Gluconat-Lösung wird auf eine erhöhte Temperatur zwischen 30 und 90°C gebracht, vorzugsweise auf etwa 50–70°C, und man stellt mit einer Säure, vorzugsweise einer Mineralsäure, insbesondere Salzsäure, auf einen pH-Wert von 4–6, insbesondere etwa 4,5–5,5 ein.

Zur Oxidation der Gluconsäure lässt man dann eine relativ konzentrierte Hypochlorit-Lösung zufliessen, wobei man durch gleichzeitige Säurezugabe, vorzugsweise Salzsäure, den pH-Wert der Reaktionslösung konstant hält. Trotz der bekannten Instabilität von Hypochlorit, das sich besonders in saurer Lösung sehr leicht unter Chlor-Entwicklung zersetzt oder in neutraler Lösung zu Chlorat und Chlorid disproportioniert, kann offenbar die Oxidationsreaktion mit diesen Zerfallsreaktionen konkurrieren, und es wird bei der erfindungsgemässen Reaktion sogar erstaunlicherweise auch in bezug auf eingesetztes Hypochlorit eine bessere Ausbeute als bei der bei tiefer Temperatur und niedriger Konzentration ablaufenden bekannten Reaktion erreicht. In der Regel werden 1,1–1,2 Äquivalente des Oxidationsmittels eingesetzt.

Die Hypochlorit-Lösung wird so zudosiert, dass innerhalb von etwa 10–60 Minuten, vorzugsweise etwa 30 Minuten, die Zugabe beendet ist. Selbstverständlich kann das Zudosieren auch über einen längeren Zeitraum erfolgen, was bei sehr grossen Ansätzen auch durchaus zwingend sein kann. In der Regel wird man jedoch versuchen, mit einer möglichst kurzen Zeit auszukommen. Man rührt danach noch kurz bis überschüssiges Hypochlorit verschwunden ist und kann dann D-Arabinose auf übliche Weise isolieren. Selbst bei Einsatz der aus D-Glucose gewonnenen Fermentations-Lösung wird eine Ausbeute an D-Arabinose von etwa 70–75%, bezogen auf D-Glucose, erreicht.

Zur Isolierung der D-Arabinose ist bekannt, die Reaktionslösung einzuengen, wobei die Hauptmenge an Kochsalz ausfällt und abgetrennt werden kann, und danach die Lösung durch Ionenaustausch von der Restmenge an ionischen Bestandteilen zu befreien.

Eine besonders vorteilhafte Methode, die insbesondere auch Gegenstand der vorliegenden Erfindung ist, ist die Abtrennung von ionischen Bestandteilen der Reaktionslösung mit Hilfe der Elektrodialyse. Dieses Verfahren bietet wesentliche verfahrenstechnische Vorteile. So werden z.B. erhebliche Mengen an Chemikalien eingespart, die sonst zur Regenerierung der Ionenaustauscher benötigt werden. Ein überraschend vorteilhafter Effekt ist auch der, dass bei der Elektrodialyse die ionischen Bestandteile mitsamt einer Hydrathülle durch die Austauschermembran wandern und so gleichzeitig eine erhebliche Menge Wasser aus der Reaktionslösung entfernt wird. Da zur Gewinnung der Arabinose die Reaktionslösung ohnehin aufkonzentriert werden muss, werden dadurch erhebliche Mengen an Energie eingespart. Die eingeengte Lösung wird dann z.B. mit Methanol ausgerührt, wobei man in hoher Ausbeute sehr reine D-Arabinose erhält. Die dabei schon sehr hoch liegende Gesamtausbeute kann noch gesteigert werden, wenn das zurückgewonnene Gluconat erneut eingesetzt wird.

Die so erhaltene D-Arabinose kann nach einem an sich aus Chemical Abstracts, Band 77, Zusammenfassung Nr. 126 942 S bekannten Verfahren unter Katalyse durch Molybdänsäure oder eine andere Molybdän(VI)-Verbindung zu einem Epimerengemisch umgesetzt werden, das D-Arabinose und D-Ribose im Verhältnis von etwa 3:1 enthält, daneben aber auch Pentosen wie D-Lyxose und D-Xylose sowie andere Nebenprodukte. Es ist auch bekannt, dass eine Auftrennung dieser Stoffe mit chromatografischen Methoden erfolgen kann. Für die grosstechnische Herstellung von D-Ribose eignen sich solche Verfahren wegen des erheblichen Arbeits- und Investitionsaufwands, der damit verbunden ist, jedoch wenig. Obwohl die für die weitere Umsetzung zu Riboflavin allein erwünschte D-Ribose nur den geringeren Teil des Epimerengemisches ausmacht, hat sich überraschenderweise doch dieser Weg zur Ribose im Gesamtverfahren als sehr vorteilhaft erwiesen. Einmal kann die als Hauptbestandteil des Epimerengemisches vorliegende D-Arabinose sehr einfach fast vollständig aus dem Epimerengemisch abgetrennt werden. Zum anderen wurde gefunden, dass überraschenderweise das bei der mit Molybdänsäure oder anderen Molybdän(VI)-Verbindungen katalysierten Epimerisierung von D-Arabinose anfallende Epimerengemisch, das nach der Grobabtrennung von D-Arabinose aus den Pentosen D-Ribose, D-Arabinose, D-Xylose, D-Lyxose und weiteren Nebenprodukten besteht, mit Vorteil direkt in die katalytische Hydrierung in Gegenwart von Nitroxylol oder Xylidin eingesetzt werden kann. Dabei kann überraschenderweise aus dem erhaltenen Reaktionsgemisch das reine N-D-Ribityl-3,4-xylidin durch Kristallisation gewonnen werden. Dies ist insofern überraschend, als aufgrund der sehr grossen konstitutionellen Ähnlichkeit der gebildeten Pentityl-xylidine zu erwarten war, dass ein Gemisch der Ribityl-, Arabityl-, Xylityl- und Lyxityl-xylidine auskristallisieren würde, aus dem das allein erwünschte Ribityl-xylidin nur durch aufwendige Reinigungsschritte in reiner Form gewonnen werden könnte. Auch dieses Verfahren stellt daher einen besonders vorteilhaften erfinderischen Aspekt der vorliegenden Anmeldung dar. Der Einsatz von Rohribose in den Hydrierungsschritt wurde auch in US-A-2 477 560 vorgeschlagen. Die Verunreinigung dort bestand jedoch im wesentlichen aus Natriumsulfat, während beim vorliegenden Verfahren ca. 25% organische Verunreinigungen enthalten sind, die aufgrund ihrer chemischen Ähnlichkeit mit D-Ribose Schwierigkeiten bei der Abtrennung des gewünschten Reaktionsproduktes erwarten liessen.

Das Verfahren läuft dabei im einzelnen so, dass die Arabinose in Wasser gelöst und bei erhöhter Temperatur mit dem Katalysator versetzt wird. Die Konzentration der Arabinose ist dabei nicht kritisch, es werden jedoch im Sinne einer guten Ausnutzung der vorhandenen Apparaturen möglichst hohe Konzentrationen verwendet, z.B. etwa 10–20gewichtsprozentige Lösungen. Es kann auch die bei der Gluconatoxidation anfallende Lösung ohne Isolierung der Arabinose verwendet werden. Diese Lösung wird auf eine Temperatur von etwa 80–100 °C erhitzt und mit etwa 1 Gew.-% Katalysator, z.B. Molybdänsäure, bezogen auf Arabinose, versetzt, wobei es vorteilhaft ist, wenn ein pH-Wert um etwa 3 eingestellt wird. Mit steigender Katalysatormenge und mit steigender Temperatur wird eine schnellere Einstellung des Epimerengleichgewichts erreicht. Bei einer Temperatur von etwa 90–95 °C und 1 Gew.-% Molybdänsäure ist nach etwa 2 Stunden der Gleichgewichtszustand erreicht, d.h. es liegt ein Gemisch der 4 Pentosen Arabinose, Ribose, Lyxose und Xylose vor neben einem gewissen Anteil von Zersetzungs- und Oxidationsprodukten. Zur Vermeidung eines übermässigen Anteils von Oxidationsprodukten kann die Apparatur bei der Reaktion mit einem Inertgas, z.B. Stickstoff, gespült werden.

Nach Beendigung der Reaktion wird der Katalysator aus der Lösung entfernt, z.B. mit Hilfe von Ionenaustauschern. Dann wird, vorzugsweise unter vermindertem Druck, eingeengt. Durch Zugabe eines niederen Alkohols, z.B. Methanol oder, vorzugsweise, Äthanol, kann die Hauptmenge der im Reaktionsgemisch vorhandenen Arabinose kristallisiert, in reiner Form abgetrennt und erneut in einem weiteren Ansatz in die Reaktion eingesetzt werden. Auch dies ist ein vorteilhafter Aspekt der vorliegenden Erfindung.

Aus der Mutterlauge, die etwa 10–20% Feststoff enthält, der zu etwa 75% aus D-Ribose, zu etwa 10% aus D-Arabinose, zu ca. 5% aus D-Xylose und D-Lyxose und zu ca. 10% aus Nebenprodukten besteht, lässt sich die Ribose durch Chromatografie über einen mit Calcium- oder Bariumionen beladenen Kationenaustauscher gewinnen.

Nach dem erfindungsgemässen Verfahren kann jedoch diese aufwendige Reindarstellung der D-Ribose unterbleiben und es kann statt dessen direkt die Mutterlauge zur katalytischen Hydrierung in Gegenwart von 4-Nitro-o-xylol oder 3,4-Xylidin eingesetzt werden. Dazu wird die Mutterlauge noch mit wässerigem Alkohol verdünnt und mit der äquivalenten Menge (bezogen auf die Gesamtmenge an Pentosen) an 4-Nitro-1,2-xylol versetzt.

Die Hydrierung selbst kann, wie in der japanischen Patentanmeldung Nr. 6665 (1964) beschrieben, mit Raney-Nickel als Katalysator bei einem Wasserstoffdruck von etwa 50–100 bar durchgeführt werden. Bei einer Hydriertemperatur von etwa 60–80 °C ist dabei die Reaktion nach etwa 30–60 Minuten beendet und nach Entfernen des Katalysators und Einengen der Lösung kristallisiert beim Abkühlen das reine N-D-Ribityl-3,4-xylidin aus.

Während bei den bisher bekannten Verfahren fast ausschliesslich die mit Nickel katalysierte Hydrierung verwendet wurde, wurde jetzt gefunden, dass die Hydrierung sehr vorteilhaft auch mit Palladium auf Kohle bei einem nur geringen Wasserstoffdruck von etwa 3 bar durchgeführt werden kann. Auch hier wird in einem Gemisch von Wasser mit einem niederen Alkohol bei erhöhter Temperatur von 50–80 °C gearbeitet, wobei die Wasserstoffaufnahme nach etwa 2–3 Stunden beendet ist. Die Ausbeute ist vergleichbar mit der bei der Hochdruckhydrierung; Vorteile liegen jedoch einmal darin, dass die Investitionen für die Hydrieranlage wesentlich geringer sind und dass zum anderen der Katalysator quantitativ zurückgewonnen und erneut eingesetzt werden kann. Eine Nachreinigung der Abwässer zur Entfernung von Nickelionen kann also unterbleiben. Nach Abtrennen des Katalysators durch Filtration kristallisiert beim Abkühlen der Lösung das reine N-D-Ribityl-3,4-xylidin in hoher Ausbeute und Reinheit.

Das N-D-Ribityl-3,4-xylidin ist als Schlüsselprodukt bei der Riboflavinsynthese anzusehen, das auch bei den anderen bekannten Riboflavinsynthesen durchlaufen wird. Die Umsetzung dieses Zwischenproduktes zum Riboflavin erfolgt bekanntlich durch Azokupplung des Ribitylxylidins mit einem Benzoldiazonium-Salz und anschliessender Umsetzung der Azoverbindung mit Barbitursäure. Diese Umsetzungen sind bekannt. Eine Literaturübersicht über diese Verfahren findet sich z. B. in Kirk-Othmer, Encyclopedia of Chemical Technology, Band 17 (1968), S. 451.

In der Regel wird dabei eine saure wässerige oder wässerig/alkoholische Lösung oder Suspension des N-D-Ribitylxylidins bei tiefer Temperatur mit Phenyldiazoniumchlorid versetzt und die gebildete Azoverbindung isoliert. Die anschliessende Umsetzung mit Barbitursäure in essigsaurer Lösung liefert unter Abspaltung von Anilin Riboflavin, das gegebenenfalls durch Auflösen in wässeriger Salzsäure, Behandeln mit Wasserstoffperoxid und Ausfällen mit Wasser noch aufgereinigt werden kann.

Die Gesamtausbeute bei dem erfindungsgemässen Verfahren, ausgehend von D-Glucose, liegt über den bei den bekannten Verfahren erzielten Ausbeuten, so dass mit dem erfindungsgemässen Verfahren ein sehr wertvolles neues Verfahren zur Herstellung von Riboflavin zur Verfügung steht.

Beispiel 1

a) Umsetzung von D-Glucose zu Natrium-D-gluconat.

In einem Fermenter werden 10 l einer sterilen wässerigen Lösung, die

1,5 kg D-Glucose $H_2O$,
10 g Cornsteep-Trockensubstanz,
10 g Ammoniumdihydrogenphosphat,
10 g Kaliumdihydrogenphosphat,
5 g Magnesiumsulfat

enthält und einen pH-Wert von 6,5 besitzt, bei einer Temperatur von 30 °C mit einer Schüttelkultur Acetobacter suboxydans ATCC 621 beimpft und unter Rühren belüftet. Durch Zudosieren von Natronlauge wird ein pH-Wert von 5,5–6,0 aufrechterhalten. Nach etwa 40 Stunden, wenn die Glucose vollständig zu Gluconat umgesetzt ist, wird gekühlt und zentrifugiert. Natriumgluconat kann durch Eindampfen der zentrifugierten Lösung in kristalliner Form mit über 90% Ausbeute gewonnen werden.

b) Umsetzung von Natrium-D-gluconat zu D-Arabinose.

Eine Lösung von 218 g Natrium-D-gluconat in 872 ml Wasser bzw. die entsprechende Menge der Fermentationslösung aus Beispiel 1a) wird auf 60 °C erhitzt und bei dieser Temperatur mit 532 ml einer Natriumhypochloritlösung mit einem Gehalt von 16% (Gew./V) an aktivem Chlor versetzt, wobei durch gleichzeitige Zugabe von etwa 80 ml konzentrierter Salzsäure der pH-Wert konstant bei 4,5–5,0 gehalten wird. Danach wird die Lösung durch Elektrodialyse von Elektrolyten befreit. Dazu wird die Lösung im Kreislauf durch eine Elektrodialysezelle mit einer wirksamen Membranfläche von 500 cm² gepumpt. Bei einer Spannung von 5 Volt und einer Stromstärke von 5 Ampere wird etwa 24 Stunden elektrolysiert bis die Arabinoselösung völlig chloridfrei und frei von ionogenen Nebenprodukten ist. Durch Eindampfen und Ausrühren des Rückstandes mit Methanol können 110 g (73%) kristalline D-Arabinose gewonnen werden.

c) Umsetzung von D-Arabinose zu D-Ribose.

Eine Lösung von 100 g D-Arabinose in 500 ml Wasser bzw. die entsprechende Menge Arabinose-Lösung aus Beispiel 1b) wird unter Spülen mit Stickstoff auf 92 °C erwärmt und mit 1 g Molybdänsäure (handelsübliche Qualität, zum Grossteil aus Ammoniummolybdat bestehend) 1–2 Stunden gerührt. Danach wird der Katalysator durch Elektrodialyse oder Ionenaustausch (stark saurer und schwach basischer Austauscher) entfernt. Die Lösung wird zu einem noch etwa 10% Wasser enthaltenden Sirup eingeengt und mit 200 ml Äthanol ausgerührt. Dabei kristallisieren 70 g D-Arabinose aus, die abgetrennt und erneut eingesetzt werden.

Die wässerig/alkoholische Mutterlauge, die etwa 21 g Di-Ribose, 3 g D-Arabinose, 3 g eines Gemisches aus D-Lyxose und D-Xylose und 3 g weiteres Produkt enthält, kann zur Reingewinnung von D-Ribose über einen mit Calcium- oder Bariumionen beladenen Kationenaustauscher chromatographiert werden.

d) Umsetzung von D-Ribose zu N-D-Ribityl-3,4-xylidin

Die alkoholische Mutterlauge aus Beispiel 1c) wird mit dem gleichen Volumen Wasser verdünnt, mit 5 g Natriumacetat und Essigsäure auf pH 5,8 eingestellt, mit 30 g 4-Nitro-o-xylol und 15 g feuchtem Raney-Nickel versetzt, auf 80 °C erwärmt und bei einem Wasserstoffdruck von 50 bar ½ Stunde hydriert. Nach Filtration vom Katalysator und Abdampfen eines Teils des Äthanols kristallisieren beim Abkühlen 30 g N-D-Ribityl-3,4-xylidin aus.

Weiteres N-D-Ribityl-3,4-xylidin kann gewonnen werden durch Eindampfen der Mutterlauge, Ausrühren des Rückstands mit 10%iger Salzsäure, wobei nur das leichtlösliche Hydrochlorid des Ribitylxylidins in Lösung geht, und Neutralisieren der vom Rückstand abgetrennten Lösung, wobei N-D-Ribityl-3,4-xylidin auskristallisiert.

e) Umsetzung von N-D-Ribityl-3,4-xylidin zu 1-D-Ribitylamino-3,4-dimethyl-6-phenylazobenzol

Eine auf −5 °C gekühlte Suspension von 616 g Anilin, 1320 ml Wasser und 1732 ml 37%iger Salzsäure wird innerhalb einer Stunde mit einer Lösung von 456 g Natriumnitrit in 1140 ml Wasser versetzt. Die so erhaltene Diazoniumsalzlösung lässt man bei einer Temperatur von max. 5 °C innerhalb 1 Stunde zu einer Suspension zulaufen, die 1532 g N-D-Ribityl-3,4-xylidin, 2200 ml Wasser, 1800 ml 100%ige Essigsäure und 470 ml 37%ige Salzsäure enthält, wobei der pH-Wert durch gleichzeitige Zugabe von 32%iger Natronlauge konstant bei 1,5 gehalten wird. Nach einigem Nachrühren stellt man mit Natronlauge pH 3,5 ein, rührt die ausgefallenen Kristalle noch einige Stunden bei Raumtemperatur und saugt ab. Man erhält 2564 g Rohprodukt, das durch Umkristallisieren aus Äthanol gereinigt werden kann.

f) Umsetzung von 1-D-Ribitylamino-3,4-dimethyl-6-phenylazobenzol zu Riboflavin

Eine Lösung von 35,9 g rohem 1-D-Ribitylamino-3,4-dimethyl-6-phenylazobenzol (erhalten nach Beispiel 1e) und 21,6 g Barbitursäure in 135 ml Dioxan und 25 ml Eisessig wird 16 Stunden gekocht. Nach dem Abkühlen wird das ausgefallene Riboflavin filtriert, mit 100 ml Wasser von 50 °C gewaschen und getrocknet, wobei man 32,7 g Rohriboflavin erhält.

Eine Aufreinigung kann so erfolgen, dass 100 g Rohriboflavin bei 50 °C in 130 ml 37%iger Salzsäure, 29 ml Wasser und 7,1 ml 35%igem Wasserstoffperoxid gelöst werden und die filtrierte Lösung mit 1144 ml Wasser eine Stunde auf 90–100 °C erhitzt wird. Nach Abkühlen wird filtriert, mit 380 ml Wasser und 160 ml Methanol gewaschen und getrocknet, wobei 88,1 g reines Riboflavin erhalten werden.

Gesamtausbeute der Stufen a–f, bezogen auf D-Glucose: 28% der Theorie.

Beispiel 2

Es wird analog Beispiel 1 gearbeitet, anstelle der unter d) beschriebenen Hydrierung wird jedoch wie folgt verfahren:

Eine Lösung von 5,243 kg Epimerengemisch aus Beispiel 1c), das etwa 60% Ribose enthält, und 5,442 kg 4-Nitro-o-xylol in 72 l 50%igem wässerigem Methanol (v/v) wird mit 0,6 kg Palladium/Kohle-Katalysator bei einer Temperatur von etwa 62 °C und einem Wasserstoffdruck von 3 bar hydriert. Nach etwa 2,5 Stunden, wenn die Wasserstoffaufnahme beendet ist, wird filtriert und das Filtrat zur Kristallisation auf 0 °C gekühlt. Nach Abschleudern, Waschen und Trocknen erhält man insgesamt 4,2 kg N-D-Ribityl-3,4-xylidin.

Der Katalysator ist nach Waschen mit Eisessig und Wasser wiederverwendbar.

**Patentansprüche**

1. Verfahren zur Herstellung von Riboflavin aus D-Glucose über N-D-Ribityl-3,4-xylidin, dadurch gekennzeichnet, dass man aufeinanderfolgend

a) D-Glucose zu D-Gluconsäure bzw. einem Alkaligluconat oxidiert,

b) das so erhaltene Gluconat bei einer Temperatur von 30 bis 90 °C mit Hilfe einer 10- bis 20gewichtsprozentigen Hypochloritlösung in D-Arabinose überführt,

c) D-Arabinose mit einer Molybdän(VI)-Verbindung zu einem auch D-Arabinose und D-Ribose enthaltenden Epimerengemisch umsetzt und aus dem Epimerengemisch die Hauptmenge der D-Arabinose abtrennt,

d) den die D-Ribose enthaltenden Rückstand in Gegenwart von 4-Nitro-o-xylol oder 3,4-Xylidin hydriert und das N-D-Ribityl-3,4-xylidin durch Kristallisation gewinnt,

e) das erhaltene N-D-Ribityl-3,4-xylidin durch Behandlung mit dem entsprechenden Diazonlumsalz zu 1-D-Ribitylamino-3,4-dimethyl-6-phenylazobenzol umsetzt und

f) diese Azoverbindung durch Umsetzung mit Barbitursäure in Riboflavin überführt.

2. Verfahren nach Anspruch 1, dadurch gekennzeichnet, dass die D-Gluconsäure bei einem pH-Wert von 4 bis 6, einer Temperatur von 30 bis 90 °C und einer Gluconsäurekonzentration von 10 bis 40 Gew.-% durch schnelles Zudosieren einer 10- bis 20gewichtsprozentigen Hypochloritlösung oxidiert wird.

3. Verfahren nach Anspruch 2, dadurch gekennzeichnet, dass das die Arabinose enthaltende Reaktionsgemisch vor der Isolierung der Arabinose durch Elektrodialyse weitgehend von ionischen Bestandteilen befreit wird.

4. Verfahren nach Anspruch 1, dadurch gekennzeichnet, dass die Hydrierung bei einer Temperatur von 50 bis 80 °C und einem Wasserstoffdruck von etwa 3 bar mit einem Palladium-Kohle-Katalysator durchgeführt wird.

## Claims

1. Process for the preparation of riboflavine from D-glucose via N-D-ribityl-3,4-xylidine, characterised in that, successively,

a) D-glucose is oxidised to D-gluconic acid or an alkali metal gluconate,

b) the gluconate thus obtained is converted into D-arabinose at a temperature of 30–90 °C with the aid of a 10 to 20% by weight hypochlorite solution,

c) D-arabinose is reacted with a molybdenum-VI compound to give an epimer mixture which contains D-ribose and also D-arabinose and most of the D-arabinose is separated off from the epimer mixture,

d) the residue containing D-ribose is hydrogenated in the presence of 4-nitro-o-xylene or 3,4-xylidine and the N-D-ribityl-3,4-xylidine is obtained by cristallisation,

e) the resulting N-D-ribityl-3,4-xylidine is converted into 1-D-ribitylamino-3,4-dimethyl-6-phenylazobenzene by treatment with the corresponding diazonium salt, and

f) this azo compound is converted into riboflavine by reaction with barbituric acid.

2. Process according to Claim 1, characterised in that the D-gluconic acid is oxidised at a pH value of 4–6, at a temperature of 30–90°C and at a gluconic acid concentration of 10 to 40% by weight, by rapidly metering in a 10 to 20% by weight hypochlorite solution.

3. Process according to Claim 2, characterised in that the reaction mixture containing arabinose is largely freed from ionic constituents by electrodialysis, before the arabinose is isolated.

4. Process according to Claim 1, characterised in that the hydrogenation is carried out at a temperature of 50–80°C and under a hydrogen pressure of about 3 bars, using a palladium-on-charcoal catalyst.

## Revendications

1. Procédé de préparation de riboflavine à partir de D-glucose via la N-D-ribityl-3,4-xylidine, caractérisé en ce qu'il comprend les étapes successives qui consistent à:

a) oxyder le D-glucose en acide D-gluconique ou en un gluconate alcalin,

b) transformer le gluconate ainsi obtenu en D-arabinose à une température de 30–90°C à l'aide d'une solution d'hypochlorite à 10–20% en poids,

c) soumettre le D-arabinose à une réaction de transformation en un mélange d'épimères contenant également du D-arabinose et du D-ribose avec un composé de molybdène(VI) et séparer la quantité principale de D-arabinose du mélange d'épimères,

d) hydrogéner le résidu contenant le D-ribose en présence de 4-nitro-o-xylène ou de 3,4-xylidine et obtenir la N-D-ribityl-3,4-xylidine par cristallisation,

e) soumettre la N-D-ribityl-3,4-xylidine obtenue à une réaction de transformation en 1-D-ribityl-amino-3,4-diméthyl-6-phénylazobenzène par traitement avec le sel de diazonium correspondant, et

f) transformer cet azo-composé en riboflavine par réaction avec l'acide barbiturique.

2. Procédé suivant la revendication 1, caractérisé en ce qu'on oxyde l'acide D-gluconique à un pH de 4–6, à une température de 30–90°C et à une concentration en acide gluconique de 10 à 40% en poids par dosage rapide d'une solution d'hypochlorite à 10–20% en poids.

3. Procédé suivant la revendication 2, caractérisé en ce que, avant l'isolation de l'arabinose, on libère essentiellement le mélange réactionnel contenant ce dernier des constituants ioniques par électrodialyse.

4. Procédé suivant la revendication 1, caractérisé en ce qu'on effectue l'hydrogénation à une température de 50 à 80°C et sous une pression d'hydrogène d'environ 3 bars avec un catalyseur de charbon palladié.